# EUROPÄISCHE PATENTSCHRIFT

(11) **EP 2 542 992 B1**
(45) Veröffentlichungstag und Bekanntmachung des Hinweises auf die Patenterteilung: **08.11.2017**
(21) Anmeldenummer: 11707397.3
(22) Anmeldetag: 03.03.2011
(51) Int. Cl.: G06F 19/00, G01N 35/00

(54) **SYSTEM FÜR EINE DATENÜBERTRAGUNG BEI DER ERMITTLUNG VON MEDIKAMENTENPARAMETERN**
SYSTEM FOR DATA TRANSMISSION IN DETERMINATION OF MEDICAMENT PARAMETERS
SYSTÈME DE TRANSMISSION DE DONNÉES DANS LA DETERMINATION DE PARAMETRES DE MÉDICAMENTS

(30) Priorität: 24.03.2010 DE 102010012733; 05.03.2010 DE 102010010567
(43) Veröffentlichungstag der Anmeldung: 09.01.2013
(73) Patentinhaber: B. Braun Melsungen AG, 34212 Melsungen (DE)
(72) Erfinder: MAIER, Hans-Otto, 34212 Melsungen (DE); DÖNHOFF, Torsten, USA RIVER (TZ); SCHMOLL, Horst, 34302 Guxhagen (DE); PAETZOLD, Matthias, 34212 Melsungen (DE)
(74) Vertreter: Winter, Brandl, Fürniss, Hübner, Röss, Kaiser, Polte - Partnerschaft mbB
(86) Internationale Anmeldenummer: PCT/EP2011/053244
(87) Internationale Veröffentlichungsnummer: WO 2011/107567

(56) Entgegenhaltungen:
- WO-A1-2005/050497
- WO-A2-2008/083313
- JP-A- 2009 273 502
- US-A1- 2006 009 949
- US-A1- 2006 229 551

## Beschreibung

Die Erfindung betrifft ein System zur Überwachung von jeweils mindestens einem Blutparameter des Blutes von verschiedenen Patienten gemäß dem Oberbegriff des Patentanspruchs 1.

Herkömmlicherweise werden Patienten, die insbesondere auf der Intensivstation liegen, mit Medikamenten und gegebenenfalls künstlicher Nahrung mittels einer oder mehrerer Zuführeinrichtungen, beispielsweise intravenös oder mittels Magensonde, versorgt. Beispielsweise kann die Zuführeinrichtung eine Insulinzuführeinrichtung bzw. eine Infusionspumpe sein, die den innerhalb des Blutkreislaufes des Patienten vorhandenen Insulinwert auf ein vorbestimmbares Niveau in Reaktion auf einen zuvor gemessenen Blutglukosewert im Blutkreislauf des Patienten hält. Ebenso können Zuführeinrichtungen für mindestens einen Nahrungswert einer mit mindestens einer Nahrungszuführeinrichtung dem Blutkreislauf direkt oder indirekt zugeführten Nahrung dem Patienten verabreicht werden.

Derartige Zuführeinrichtungen, auch wenn sie in ein System zur Verabreichung von Medikamenten und/oder Nahrungswerten integriert sind, erfordern bisher die durch einen Arzt oder ein weiteres klinisches Personal zu erfolgende Eingabe von Werten, die der Zuführung mittels der Zuführeinrichtung zugrunde liegen. Beispielsweise können hier Mengenwerte, Zeitabstände, in welchen die Zuführung erfolgen soll, intervallartige Zuführungen, etc als Basis für die anschließend zu erfolgende Zuführung von beispielsweise Insulin eingegeben werden.

Dieser Zuführung vorausgehend ist eine zumeist von Hand durchgeführte Blutentnahme bei den Patienten, welche die Zwischenschaltung von klinischem Personal erfordert. Ebenso ist weiteres klinisches Personal erforderlich, welches die notwendigen Fachkenntnisse zu den Eingabefunktionen der Zuführeinrichtung, wie der Infusionspumpe hat, um anschließend diese Zuführung durchzuführen.

Häufig ergibt sich hieraus das Problem, dass durch die Zwischenschaltung des klinischen Personals fehlerhafte Vorgehensweisen stattfinden. Beispielsweise ist es denkbar, dass eine Blutprobe dem falschen Patienten zugeordnet wird und somit die Gefahr der Verabreichung falscher Medikamente sowohl bei der Eingabe von Daten in die Zuführeinrichtung als auch bei der Blutentnahme und auch bei der Durchführung einer Blutanalyse besteht.

Hinzu kommt, dass derartige Zuführeinrichtungen, wie Infusionspumpen, eine Förderrate aufweisen, die als Volumen pro Zeiteinheit (ml/h) dargestellt ist. Dem gegenüber wird in der Medizin für eine zugeführte Medikamentenlösung die Dosiseinheit verwendet. Demzufolge ist es erforderlich, dass die Dosiseinheit in eine Förderrate der Pumpe umgerechnet wird, welches die Aufgabe des behandelnden Arztes ist. Nachteilhaft bei einer derartigen Umrechnung können häufig Rechenfehler sein, die zu Falscheingaben der Förderrate und damit zu einer unrichtigen Verabreichung von Insulin für den Patienten führt.

Denkbar ist auch, dass bei einer Infusionspumpe die Eingabe der Dosiseinheit ermöglicht wird. Allerdings erfordert dies die Berücksichtigung von Angaben über die Konzentration des Wirkstoffes des zu verabreichenden Medikamentes und über die Art des Medikamentes. Sowohl bei der Angabe der Konzentration des Wirkstoffes als auch bei der Eingabe der Dosiseinheit und der Umrechnung derjenigen in die Förderrate werden bisher ausschließlich die Hauptwirkstoffe des Medikamentes berücksichtigt. Dies ist häufig ausreichend, sofern lediglich oder vorrangig ein spezifisches Medikament verabreicht werden soll.

Zuführeinrichtungen bzw. Fördervorrichtungen für die Zuführung eines Medikamenten-Lösungsgemisches in einem Körper sind mehrfach bekannt. Beispielsweise sind Vorrichtungen/Systeme mit einer Mehrzahl an Infusions- und/oder Spritzenpumpen, wovon jeder eine Lösung mindestens einem spezifischen Medikamentenwirkstoff einem Körper zuführt und hierdurch ein Medikamenten-Lösungsgemisch entsteht, bekannt.

Derartige Infusionspumpen-Systeme werden häufig bei Patienten, die einer intensiven, medizinischen Behandlung bedürfen, verwendet. Hierbei weisen die Infusionspumpen die Eigenschaften der kontinuierlichen und genauen Dosierung der Medikation in ihrer Zuführung auf. Um eine optimierte Abstimmung dieser Pumpen in ihrer Dosierung zu erreichen, werden die Pumpen in einem gemeinsamen Ordnungssystem integriert, welches üblicherweise eine zentrale Steuereinheit, eine Bedienungseinheit und eine Alarmierungseinheit aufweist.

Die datentechnische Verbindung mehrerer Pumpen und/oder Steuereinheiten kann auch im Rahmen eines Servers zusammengefasst sein. Dies ermöglicht gegebenenfalls in einer zusätzlichen Recheneinheit/einem medizinischen Computer, dass sogar eine zweistellige Anzahl an verschiedenen Medikamenten auf einem Körper abgestimmt und genau dosiert zugeführt werden kann. Die Daten werden drahtgebunden oder per Wireless-LAN an die verschiedenen Infusionspumpen verteilt.

Auch im Falle der Verwendung eines Servers ist häufig das Problem, dass eine richtige Zuordnung der ermittelten Medikamente mit den dazugehörigen Medikamentenparametern zu der richtigen Infusionspumpe oder Mehrzahl an Infusionspumpen, die zu dem Patienten mit den ermittelten Blutparameterwerten zugehörig ist, durch Zwischenschaltung von klinischem Personal nicht garantiert ist.

Ebenso kann mittels des gemeinsamen Servers eine Vielzahl an Pumpen bzw. Fördervorrichtungen angesteuert werden, die bei verschiedenen Patienten angeordnet sind und zur Versorgung dieser verschiedenen Patienten dienen.

WO 2005 / 050497 A1 beschreibt ein medizinisches Behandlungsmanagementsystem, bei welchem sowohl der Patient als auch Behandlungsvorrichtungen, wie z.B. Spritzen zur Entnahme von Blut und zum Zuführen von behandeltem Blut mit Datenträgern in Form von RFID-Chips versehen werden. Eine Vergleichseinheit führt einen Vergleich zwischen den jeweiligen Datensätzen durch und erlaubt einer Behandlungsvorrichtung die Durchführung einer Behandlung lediglich bei Übereinstimmung der Datensätze.

Weiterhin ist aus der US 2006/0229551 A1 ein System zur Identifizierung von Patienten und zum Medikamentenmanagement bekannt, bei welchem Patienten- und Medikamentendaten jeweils mit gespeicherten Daten abgeglichen werden.

Zudem offenbart die japanische Patentschrift JP 2009 273502 A eine Vorrichtung zur Überwachung der Medikation eines Patienten, bei welcher Patienten- und Medikamentendaten jeweils mit in einer Datenbank gespeicherten Daten abgeglichen werden.

Demzufolge ist es Aufgabe der Erfindung, ein System zur Überwachung von jeweils mindestens einem Blutparameter des Bluts von verschiedenen Patienten zur Verfügung zu stellen, bei dem eine fehlerfreie Zuordnung berechneter Medikamentenparameter zu den jeweiligen Zuführeinrichtungen sichergestellt ist.

Diese Aufgabe wird durch die Merkmale des Anspruches 1 gelöst.

Ein wesentlicher Punkt der Erfindung ist es, dass bei einem System zur Überwachung von jeweils mindestens einem Blutparameter des Bluts von verschiedenen Patienten mit einer Mehrzahl an Zugangseinrichtungen zum Aufbau von jeweils mindestens einem Zugang zum Blut von jedem Patienten durch dessen Haut, einer Mehrzahl an Entnahmeeinrichtungen jeweils zum Entnehmen einer Blutmenge von jedem Patienten zum Erhalten von jeweils mindestens einer Blutprobe, mindestens einer Blutanalyseeinrichtung zum Analysieren der Blutprobe hinsichtlich vorbestimmter Parameter des Blutes und zur Erzeugung von einzelnen Blutparameterdatensätzen, einer für eine Mehrzahl an Blutparameterdatensätzen verschiedener Patienten gemeinsam verwendbare Berechnungseinrichtung zum Berechnen von Datensätzenmedikamentenparametern der dem Patienten zu verabreichenden Medikamente auf Basis der einzelnen Blutparameterdatensätze und einer Mehrzahl an Zuführeinrichtungen zum Zuführen des jeweiligen Medikamentes mit den berechneten Medikamentenparametern eine Zuordnungseinrichtung zur Verfügung gestellt wird, die zum Zuordnen jeweils einer Identifikationskennung zur Identifizierung eines Patienten und eine Zeitabschnittskennung zur Angabe eines Zeitabschnittes, in dem die Blutentnahme stattfand, zu jedem Datensatz der von der Berechnungseinrichtung berechneten Medikamentenparameter eines Patienten durchführt. Eine derartige Zuordnungseinrichtung ist vorteilhaft innerhalb der Berechnungseinrichtung angeordnet.

Eine derartige Zuordnungseinrichtung wird dafür verwendet, dass die Blutparameter-Datensätze, wie sie von der Blutanalyseeinrichtung durch die Berechnungseinrichtung erhalten werden, zuverlässig der richtigen Identifikationskennung und der richtigen Zeitabschnittskennung zugeordnet werden, um sicherzustellen, dass die sich daraus ergebenden Medikamentenparameterdatensätze der richtigen Zuführeinrichtung, welches eine Infusionspumpe zur Verabreichung von Insulin in Antwort auf einen zuvor gemessenen Blutglukosespiegel sein kann, zugesendet werden. Auf diese Weise ist ausgeschlossen, dass berechnete Medikamentenparameterdatensätze an die falschen Infusionspumpen übertragen werden und somit ein Patient eine falsche Verabreichung erhält, wie es beispielsweise bei Zwischenschaltung von medizinischem Personal für die Eingabe von Datensätzen an den Infusionspumpen der Fall sein kann.

Zudem kann über die Zuordnung von einer Zeitabschnittskennung sichergestellt werden, dass bei der Entnahme mehrer Blutproben von dem gleichen Patienten gewährleistet ist, dass nicht vorausgegangene berechnete Medikamentenparameterdatensätze an die dem Patienten zugeordnete Infusionspumpe gesendet werden, sondern nur derjenige Medikamentenparameterdatensatz, der zuletzt in Verbindung mit der Identifikationskennung zu dem ausgewählten Patienten berechnet worden ist.

Vorteilhaft ist jeder Datensatz der Medikamentenparameter für einen Patienten zusammen mit der Identifikationskennung und der Zeitabschnittskennung jeweils als gemeinsamer Datensatz leitungsgebunden oder leitungslos von der Berechnungseinrichtung an eine oder mehrere der Zuführeinrichtungen übertragbar. Aufgrund der leitungslosen Übertragung kann eine räumliche Trennung von der Berechnungseinrichtung, die innerhalb eines gemeinsamen Servers angeordnet sein kann, und den einzelnen Zuführeinrichtungen, die in der Regel als Infusionspumpen in verschiedenen Zimmern eines Krankenhauses direkt vor Ort zu dem Patienten angeordnet sind, bestehen.

Mindestens eine Identifikationselementen-Erzeugungseinrichtung wird vorzugsweise dafür verwendet, dass jede Blutprobe unmittelbar nach dem Entnehmen der Blutmenge und vor dem Zuführen zu der Blutanalyseeinrichtung die Identifikationskennung und/oder die Zeitabschnittskennung in Form von einem Barcode, einem Datenmatrix-Code und/oder Transponder auf der die Blutprobe enthaltenden Entnahmeeinrichtung zuordenbar ist.

Weitere vorteilhafte Ausführungsformen ergeben sich aus den Unteransprüchen und der nachfolgenden Beschreibung in Verbindung mit der Zeichnung. Hierbei zeigen:
Fig. 1 In einer schematischen Darstellung das erfindungsgemäße System gemäß einer Ausführungsform der Erfindung in seinem Funktionsablauf; und
Fig. 2 In einem Flussdiagramm das erfindungsgemäße Verfahren gemäß einer Ausführungsform der Erfindung.

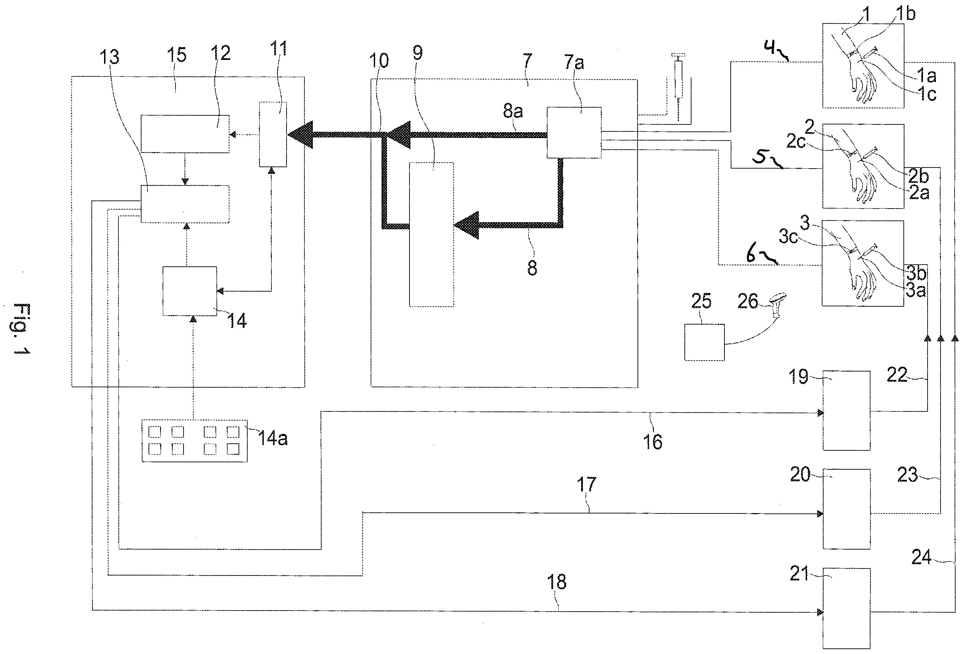

In Fig. 1 ist in einer schematischen Darstellung das erfindungsgemäße System in einer Ausführungsform der Erfindung gezeigt. Wie der Darstellung gemäß Fig. 1 zu entnehmen ist, wird bei insgesamt drei stationär liegenden Patienten 1, 2 und 3 mittels eines Blutentnahmesystems (z.B. einer Spritze 1b, 2b und 3b, die jeweils eine Nadel 1a, 2a und 3a aufweisen) Blut entnommen.

Jeder Patient hat an seinem Arm ein Armband 1c, 2c und 3c, auf dem patientenspezifische Daten, beispielsweise mittels eines Barcodes, eines Datamatrix-Codes oder eines Transponders angeordnet sind. Dies können beispielsweise die Identifikationskennungen des Patienten sein.

Sobald eine Blutprobe mittels des Blutentnahmesystems 1b, 2b und 3b entnommen worden ist, werden diese Probenbehälter (z.B. Spritze) gemäß den dargestellten Transportwegen 4, 5 und 6 zu einer Blutanalyseeinrichtung 7 transportiert.

Zeitgleich oder zuvor werden mittels eines Barcode- oder Datamatrix-Code-Lesers 26 oder einer Leseeinheit für Transponder die Kennungen auf dem Armband 1c, 2c und 3c des Patienten 1 , 2, 3 ausgelesen und mittels einer Barcode- oder Datamatrix-Code-Erzeugungseinrichtung 25 oder mittels einer Schreibeinheit für Transponder ein entsprechender Barcode oder ein Datamatrix-Code ausgedruckt bzw. ein Transponder beschrieben. Dieser Barcode bzw. Datamatrix-Code oder dieser Transponder werden auf die jeweiligen Probenbehälter (z.B. Spritze) außenseitig aufgeklebt.

Bei Ankunft bei der Blutanalyseeinrichtung 7 werden diese Barcodes, Datamatrix-Codes und/oder der Transponder mittels einer Leseeinheit 7a ausgelesen und zugleich die Blutproben in die Blutanalyseeinrichtung eingegeben. Ein anschließender Datentransfer von der Leseeinheit 7 zu einer Analyseeinheit 9 innerhalb der Blutanalyseeinrichtung 7, wie er durch Bezugzeichen 8 wiedergegeben wird, führt dazu, dass die Identifikationskennung und die Zeitabschnittskennung an die Analyseeinheit und gemäß Bezugszeichen 10 an eine Zuordnungseinrichtung 13 weitergegeben werden, wobei zugleich innerhalb der Analyseeinheit 9 eine Analysierung der Blutprobe zur Feststellung vorbestimmbarer Blutparameter stattfindet.

Die von der Analyseeinheit erstellten Blutparameterdatensätze werden mittels einer gemeinsamen Leitung 10 an eine Empfangseinheit 11 einer Berechnungseinrichtung 15 leitungslos oder leitungsgebunden übertragen. Diese Empfangseinheit erhält ebenso - wie mit dem Bezugszeichen 8a dargestellt - Daten zu der Identifikationskennung und der Zeitabschnittskennung. Sowohl die Identifikationskennungsdaten als auch die Zeitabschnittskennungsdaten und die Blutparameterdatensätze werden von der Empfangseinheit 11 an die Berechnungseinheit 12 weitergegeben, welche aus den übermittelten Blutparameterdatensätzen Medikamentenparameterdatensätze berechnet, welche zur Verabreichung der Medikamente zu ermitteln sind.

Anschließend leitet die Berechnungseinheit 12 die berechneten Medikamentenparameterdatensätze an eine Zuordnungseinrichtung 13 weiter, welche dazu geeignet ist, jedem berechneten Medikamentenparameterdatensatz eine der übermittelten Identifikationskennungen und/oder Zeitabschnittskennungen zuzuordnen.

Zusätzlich kann mittels einer Tastatur 14a oder einer ähnlichen Eingabeeinrichtung weitere Parameter in eine Speichereinheit 14 eingegeben werden oder auch durch Kommunikation mit der Empfangseinheit 11 die entsprechenden übertragenen Identifikationskennungen und Zeitabschnittskennungen zu dem jeweiligen Blutparameterdatensatz geändert werden. Dies wird dann ebenso der Zuordnungseinrichtung 13 für eine richtige Zuordnung zu dem jeweils berechneten Medikamentenparameterdatensatz weiter übermittelt.

Gemäß den Bezugszeichen 16, 17 und 18 findet anschließend eine Übertragung der gemeinsamen Datensätze, die sich jeweils aus einem Datensatz der berechneten Medikamentenparameter und einer Identifikationskennung und/oder einer Zeitabschnittskennung zusammensetzen, an anhand der Identifikationskennung und der Zeitabschnittskennung ausgewählten zugehörigen Zuführeinrichtungen 19, 20 und 21 statt, die als Infusionspumpen als Medikament, zum Beispiel Insulin, dem jeweils zugehörigen Patienten 1, 2, 3 verabreichen. Dies wird mittels der Bezugszeichen 22, 23 und 24 dargestellt.

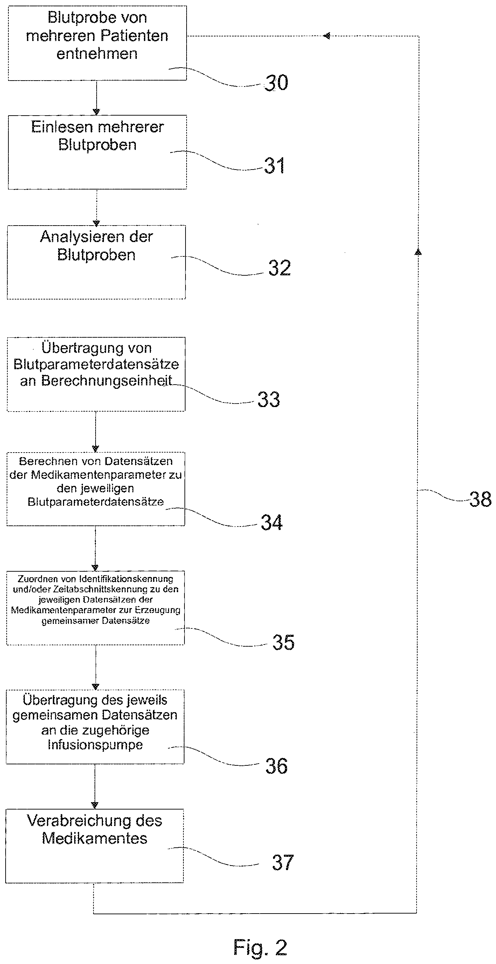

In dem Flussdiagramm gemäß Fig. 2 wird ein Verfahren gemäß einer Ausführungsform gezeigt.

Dieser Darstellung ist zu entnehmen, dass in einem anfänglichen Schritt 30 Blutproben von mehreren Patienten entnommen werden, die anschließend in der Blutanalyseeinrichtung eingelesen werden (Schritt 31).

Nach einem Analysieren der Blutprobe gemäß Schritt 32 findet in Schritt 33 eine Übertragung von Blutparameterdatensätzen, die sich aus der Analysierung der Blutgruppen ergeben, an die Berechnungseinrichtung statt.

In der Berechnungseinrichtung werden Datensätze der Medikamentenparameter zu den jeweiligen Blutparameterdatensätzen gemäß Schritt 34 berechnet, um anschließend in Schritt 35 eine Zuordnung der Identifikationskennung und der Zeitabschnittskennung zu den jeweiligen Datensätzen der Medikamentenparameter zur Erzeugung von gemeinsamen Datensätzen erfolgen zu lassen.

Nach einer Übertragung des jeweils gemeinsamen Datensatzes an die zugehörige Infusionspumpe gemäß Schritt 36 findet eine Verabreichung des Medikamentes, Schritt 37 statt.

Mit Bezugszeichen 38 wird dargestellt, dass nach einem vorbestimmbaren Zeitabschnitt wiederum eine Blutentnahme gemäß Schritt 30 stattfindet und somit ein geschlossener Kreislauf existiert.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Patient 1
- 1a: Nadel Patient 1
- 1b: Spritze Patient 1
- 1c: Armband Patient 1
- 2: Patient 2
- 2a: Nadel Patient 2
- 2b: Spritze Patient 2
- 2c: Armband Patient 2
- 3: Patient 3
- 3a: Nadel Patient 3
- 3b: Spritze Patient 3
- 3c: Armband Patient 3
- 4: Transportweg
- 5: Transportweg
- 6: Transportweg
- 7: Blutanalyseeinrichtung
- 7a: Leseeinheit
- 8: Datentransfer
- 9: Analyseeinheit
- 10: gemeinsame Leitung
- 11: Empfangseinheit
- 12: Berechnungseinheit
- 13: Zuordnungseinrichtung
- 14: Speichereinheit
- 14a: Tastatur
- 15: Berechnungseinrichtung
- 16: Senden der Medikamentenparameter an die Infusionspumpe
- 17: Senden der Medikamentenparameter an die Infusionspumpe
- 18: Senden der Medikamentenparameter an die Infusionspumpe
- 19: Zuführeinrichtung
- 20: Zuführeinrichtung 21 Zuführeinrichtung
- 22: Verabreichen der richtigen Dosis des Insulins
- 23: Verabreichen der richtigen Dosis des Insulins
- 24: Verabreichen der richtigen Dosis des Insulins
- 25: Barcode- oder Datamatrix-Code-Erzeugungseinheit
- 26: Datamatrix-Code-Leser
- 30: Entnehmen einer Blutmenge
- 31: Einlesen der Blutproben in der Blutanalyseeinrichtung
- 32: Analysieren der Blutprobe
- 33: Übertragung der Blutparameterdatensätze
- 34: Berechnen der Medikamentenparameter
- 35: Zuordnung der Kennungen der Medikamentenparameter
- 36: Übertragung der Medikamentenparameter an die Infusionspumpen
- 37: Verabreichung des Medikamentes
- 38: Kreislauf des Verfahrens

## Patentansprüche

1. System zur Überwachung von jeweils mindestens einem Blutparameter des Blutes von verschiedenen Patienten (1, 2, 3) umfassend:
- eine Mehrzahl an Zugangseinrichtungen (1a, 2a, 3a) zum Aufbau von jeweils mindestens einem Zugang zum Blut von jedem Patienten (1, 2, 3) durch dessen Haut;
- eine Mehrzahl an Entnahmeeinrichtungen (1b, 2b, 3b) zum jeweiligen Entnehmen einer Blutmenge von jedem Patienten (1-3) zum Erhalten von jeweils mindestens einer Blutprobe;
- eine Blutanalyseeinrichtung (7, 9) zum Analysieren der Blutproben hinsichtlich vorbestimmbarer Parameter des Blutes und zur Erzeugung von einzelnen Blutparameterdatensätzen;
- eine für eine Mehrzahl an Blutparameterdatensätzen verschiedener Patienten gemeinsam verwendete Berechnungseinrichtung (15) zum Berechnen von Datensätzen an Medikamentenparametern der den jeweiligen Patienten (1-3) zu verabreichenden Medikamente auf Basis der einzelnen Blutparameterdatensätze, wobei die Berechnungseinrichtung (15) eine Empfangseinheit (11) aufweist, an welche die von der gemeinsamen Blutanalyseeinrichtung (7,9) erstellten Blutparametersätze mittels einer gemeinsamen Leitung (10) leitungsgebunden übertragen werden, wobei die Empfangseinheit (11) zudem Daten zu der Identifikationskennung und der Zeitabschnittskennung über die gemeinsame Leitung (10) erhält;
- eine Mehrzahl an Zuführeinrichtungen (19-21) zum Zuführen des jeweiligen Medikamentes zu den verschiedenen Patienten; wobei jede der Mehrzahl an Zuführungseinrichtungen jeweils fest einem Patienten zugeordnet ist, und
- eine Zuordnungseinrichtung (13), welche jeweils eine Identifikationskennung zur Identifizierung eines Patienten und eine Zeitabschnittskennung zur Angabe eines Zeitabschnittes, in dem die Blutentnahme stattfand, zu jedem Datensatz der von der Berechnungseinrichtung (15) berechneten Medikamentenparameter eines Patienten (1-3) zuordnet;
**dadurch gekennzeichnet, dass**
jeder Datensatz der Medikamentenparameter für einen jeweiligen Patienten zusammen mit der Identifikationskennung und der Zeitabschnittkennung jeweils als gemeinsamer Datensatz leitungsgebunden oder leitungslos von der Berechnungseinrichtung (15) an zumindest eine anhand der Identifikationskennung und der Zeitabschnittskennung ausgewählte zugehörige Zuführeinrichtung (19, 20, 21) übertragen wird; und
die zumindest eine zugehörige Zuführeinrichtung (19, 20, 21) das jeweilige Medikament mit einem Mengenwert entsprechend dem gemeinsamen Datensatz dem jeweiligen Patienten zuführt.

2. System nach Anspruch 1, wobei die Zuordnungseinrichtung (13) in der Berechnungseinrichtung (15) angeordnet ist.

3. System nach einem der vorangegangenen Ansprüche, wobei mindestens eine Identifikationselementen-Erzeugungseinrichtung (25), mittels welcher jede Blutprobe unmittelbar nach dem Entnehmen der Blutmenge und vor dem Zuführen zu der Blutanalyseeinrichtung (7, 9) die Identifikationskennung und/oder die Zeitabschnittskennung in Form von einem Barcode, einem Datamatrix-Code und/oder einem Transponder auf der die Blutprobe enthaltenen Entnahmeeinrichtung zuordenbar ist.

4. System nach einem der vorangegangenen Ansprüche, wobei die Blutanalyseeinrichtung (7, 9) eine Leseeinheit (7a) zum Lesen des Barcodes, des Datamatrix-Codes und/oder des Transponders aufweist.

5. System nach Anspruch 4, wobei die Blutanalyseeinrichtung (7, 9) mit der Berechungseinrichtung (15) und diese mit den Zuführeinrichtungen (19-21) für einen Datentransfer verbunden ist.

6. System nach einem der vorangegangenen Ansprüche, wobei die Datensätze der Medikamentenparameter verschlüsselt sind.

7. System nach einem der vorangegangenen Ansprüche, wobei die Entnahmeeinrichtung eine Spritze oder ein Blutentnahmeröhrchen ist, deren Blutaufnahmebehälter oder ein damit ein verbundener Blutaufnahmebehälter außenseitig mit dem Barcode, dem Datamatrix-Code und/oder dem Transponder verbunden ist.

8. System nach einem der Ansprüche 1 bis 6, wobei innerhalb des gemeinsamen Datensatzes der Datensatz der Medikamentenparameter mit den Daten der Identifikationskennung und/oder der Zeitabschnittskennung frequenz- und/oder amplitudenmoduliert ist.

9. System nach einem der Ansprüche 1 bis 6, wobei der gemeinsame Datensatz binärcodiert vorliegt.

## Claims

1. System for monitoring at least one blood parameter of the blood of different patients (1, 2, 3) comprising:
- a multiplicity of access devices (1a, 2a, 3a) each to create at least one access to the blood of each patient (1, 2, 3) through his or her skin;
- a multiplicity of extraction devices (1b, 2b, 3b) each to extract a blood quantity from each patient (1-3) to obtain in each case at least one blood specimen;
- a blood analysis device (7, 9) to analyse the blood specimens in relation to predefinable parameters of the blood and to generate individual blood parameter data sets;
- a calculation device (15) which is used in common for a multiplicity of blood parameter data sets of different patients to calculate data sets of medicament parameters of the medicaments to be administered to the respective patients (1, 2, 3) on the basis of the individual blood parameter data sets, wherein the calculation device (15) comprises a receiver unit (11) to which the blood parameter data sets produced by the common blood analysis device (7, 9) are transmitted hard-wired by means of a common line (10), wherein the receiver unit (11) furthermore receives data on the identification code and period of time code via the common line (10);
- a multiplicity of supply devices (19-21) to supply the respective medicament to the different patients (1-3); wherein each one of the multiplicity of supply devices is fixedly allocated to a respective patient, and
- an association device (13) which allocates in each case an identification code to identify a patient and a period of time code to specify a period of time in which the blood specimen was taken, to each data set of the medicament parameters calculated by the calculation device (15) of the patient (1-3);
**characterised in that**
each data set of the medicament parameters for the respective patient, together with the identification code and period of time code, is transmitted as a common data set by hard-wired link or wireless from the calculation device (15) to at least one respective supply device (19, 20, 21) selected on the basis of the identification code and the period of time code; and
the at least one respective supply device (19, 20, 21) supplies a corresponding amount of the respective medicament according to the common data set to the respective patient.

2. System according to claim 1, wherein the association device (13) is arranged in the calculation device (15).

3. System according to one of the preceding claims, wherein at least one identification element generating device (25), by means of which, immediately after taking of the blood quantity and before supply to the blood analysis device (7, 9), each blood specimen can be associated with the identification code and/or the period of time code in the form of a barcode, a data matrix code and/or a transponder on the extraction device containing the blood specimen.

4. System according to one of the preceding claims, wherein the blood analysis device (7, 9) comprises a read unit (7a) to read a barcode, data matrix code and/or transponder.

5. System according to claim 4, wherein the blood analysis device (7, 9) is connected with the calculation device (15) which is connected with the supply devices (19-21) for data transfer.

6. System according to one of the preceding claims, wherein the data sets of the medicament parameters are encrypted.

7. System according to one of the preceding claims, wherein the extraction device is a syringe or a blood extraction tube, a blood receiving container of which or a connected blood receiving container on the outside is connected with a barcode, data matrix code and/or transponder.

8. System according to one of claims 1 to 6, wherein within the common data set, the data set of the medicament parameters with the data of the identification code and/or the period of time code is frequency- and/or amplitude-modulated.

9. System according to one of claims 1 to 6, wherein the common data set is present in binary code.

## Revendications

1. Système pour la surveillance d'au moins un paramètre sanguin du sang de différents patients (1, 2, 3), comprenant :
- une pluralité de dispositifs d'accès (1a, 2a, 3a) pour l'établissement d'au moins un accès au sang de chaque patient (1, 2, 3) à travers leur peau ;
- une pluralité de dispositifs de prélèvement (1b, 2b, 3b) pour le prélèvement d'une quantité de sang de chaque patient (1 à 3) afin d'obtenir au moins un échantillon de sang de chacun d'eux ;
- un dispositif d'analyse sanguine (7, 9) pour l'analyse des échantillons de sang en ce qui concerne des paramètres prédéterminés du sang et afin de générer des ensembles de données de paramètres sanguins ;
- un dispositif de calcul (15), utilisé en commun pour une pluralité d'ensembles de données de paramètres sanguins de différents patients, pour le calcul d'ensembles de données de paramètres médicamenteux des médicaments à administrer aux différents patients (1 à 3) sur la base des différents ensembles de données de paramètres sanguins, le dispositif de calcul (15) comprenant une unité de réception (11) à laquelle les ensembles de paramètres sanguins créés par le dispositif d'analyse sanguine commun (7, 9) sont transmis de manière filaire au moyen d'une ligne commune (10), l'unité de réception (11) recevant en outre des données concernant un identifiant d'identification et un identifiant de période par l'intermédiaire de la ligne commune (10) ;
- une pluralité de dispositifs d'introduction (19 à 21) pour l'introduction du médicament aux différents patients ; chacun de la pluralité de dispositifs d'introduction étant assigné de manière ferme à un patient et
- un dispositif d'assignation (13), qui assigne un identifiant d'identification pour l'identification d'un patient et un identifiant de période pour l'indication d'une période à laquelle le prélèvement a eu lieu, à chaque ensemble de données des paramètres médicamenteux d'un patient (1 à 3), calculés par le dispositif de calcul (15);
**caractérisé en ce que**
chaque ensemble de donnés des paramètres médicamenteux pour un patient déterminé est transmis, conjointement avec l'identifiant d'identification et l'identifiant de période, sous la forme d'un ensemble de données commun, de manière filaire ou sans fil, par le dispositif de calcul (15) à au moins un dispositif d'introduction (19, 20, 21) correspondant sélectionné à l'aide de l'identifiant d'identification et de l'identifiant de période ; et
l'au moins un dispositif d'introduction (19, 20, 21) correspondant administre le médicament au patient avec une valeur de quantité correspondant à l'ensemble de données commun.

2. Système selon la revendication 1, le dispositif d'assignation (13) étant disposé dans le dispositif de calcul (15).

3. Système selon l'une des revendications précédentes, au moins un dispositif de génération d'éléments d'identification (25), permettant d'assigner chaque échantillon de sang, immédiatement après le prélèvement de la quantité de sang et avant l'introduction dans le dispositif d'analyse sanguine (7, 9) à l'identifiant d'identification et/ou à l'identifiant de période sous la forme d'un code barre, d'un code à matrice de données et/ou d'un transpondeur sur le dispositif de prélèvement contenant l'échantillon de sang.

4. Système selon l'une des revendications précédentes, le dispositif d'analyse sanguine (7, 9) comprenant une unité de lecture (7a) pour la lecture du code barre, du code à matrice de données et/ou du transpondeur.

5. Système selon la revendication 4, le dispositif d'analyse sanguine (7, 9) étant relié avec le dispositif de calcul (15) et celui-ci étant relié avec les dispositifs d'introduction (19 à 21) pour un transfert de données.

6. Système selon l'une des revendications précédentes, les ensembles de données des paramètres médicamenteux étant chiffrés.

7. Système selon l'une des revendications précédentes, le dispositif de prélèvement étant une seringue ou un tube de prélèvement sanguin, dont le récipient de logement du sang ou un récipient de logement du sang relié à celui-ci est relié à l'extérieur avec le code barre, le code à matrice de donnés et/ou le transpondeur.

8. Système selon l'une des revendications 1 à 6, l'ensemble de données des paramètres médicamenteux étant modulé en fréquence et/ou modulé en amplitude avec les données de l'identifiant d'identification et/ou l'identifiant de période à l'intérieur de l'ensemble de données commun.

9. Système selon l'une des revendications 1 à 6, l'ensemble de données commun étant codé de manière binaire.
